# EUROPEAN PATENT APPLICATION

(11) **EP 3 087 996 A1**
(43) Date of publication of application: **02.11.2016**
(21) Application number: 16159743.0
(22) Date of filing: 23.07.2010
(51) Int. Cl.: A61K 38/17, C07K 14/00

(54) **COMPOSITIONS AND METHODS OF USE FOR POST-RADIATION PROTECTION**

(30) Priority: 24.07.2009 US 271715 P
(62) Divisional of application: 10802970.3
(71) Applicant: Terapio Corporation, Austin, TX 78701 (US)
(72) Inventor: CUNNINGHAM, Casey, Whitehouse TX 75791 (US)
(74) Representative: Cornish, Kristina Victoria Joy

(57) **Abstract**

The present disclosure is directed to methods of administering RLIP76 or an active fragment thereof more than 24 hours after radiation exposure, wherein administration is effective for the protection and treatment of mammals exposed to radiation. In addition, compositions are disclosed including RLIP76 and other radioprotective agents, for example antioxidants.

## Description

### FIELD OF THE INVENTION

The present disclosure relates generally to exposure to radiation and more specifically to compositions comprising an RLIP76 protein moiety, and the use of such compositions in post-radiation protection of mammals.

### BACKGROUND OF THE INVENTION

Radiation damage occurs from several mechanisms, with the relative contribution of each depending upon the amount of exposure. At very high doses, radiation causes cell death from direct DNA damage resulting in complete breakage of double-helical strands of DNA (double-strand breaks, DSBs). However, as exposure levels decrease, other effects become significant. Among these are peroxidation processes due to the ionizing energy of radiation producing highly reactive free radicals, particularly associated with elemental oxygen (reactive oxygen species; ROS), even in cells whose nucleus escapes direct assault. Reactive oxygen species are toxic because of their propensity to bind to, and modify, almost anything in their path, including proteins, lipids and nucleic acids. Over time, cells with significant levels of ROS can become as equally compromised as those with direct DNA effects. ROS damage can also lead to DNA abnormalities that eventually are lethal due to genomic instability. Thus, the amount of insult sustained by a cell will depend upon the dose of radiation received and will reflect a mixture of direct DNA damage, ROS damage, and indirect DNA damage due to ROS effects. The timing, and extent, of the overall damage to an organism then depends heavily on the dose of radiation received, resulting in a progressive, non-linear relationship between survival and exposure.

Candidate treatments for radiation poisoning often have been agents that attempt to enhance the normal cellular defenses against ROS effects. Examples include free radical scavengers (such as edaravone (3 methyl-1-phenyl-2-pyrazolin-5-one), vitamin E, and the like), superoxide-dismutase analogs (such as tempol (4-hydroxy-2,2,6,6-tetramethylpiperidinyloxy)), and other agents that attempt to reduce the intracellular concentrations of ROS. These candidate treatments are designed to be administered either before or immediately after radiation exposure. Assessments of any benefit from these agents are usually designed to look for improvements in survival one month after exposure, as by that time any toxicity is the result of the indirect processes described above.

A common benchmark measurement is the dose-reduction factor (DRF), defined as the ratio of the dose of radiation that gives 50% mortality 30 days after exposure when mice are treated with a candidate agent to the dose of radiation achieving this LD50/30 when no treatment (or a control treatment) is administered. This measurement is not linear, so that small increases in DRF imply somewhat larger changes in radiation resistance. For a candidate agent to be considered promising enough for further evaluation by the Department of Defense, a DRF of 1.2 or greater is normally required. This requirement can be a significant hurdle. Both edaravone and tempol have significant effects *in in vitro* systems, but in whole animal studies manage only a reported DRF of 1.3. This limitation may arise because the cell already has defenses against free radicals, including a variety of proteins that can bind ROS before they combine with other components. But such binding does not confer complete protection in that the complexed proteins are themselves toxic to the cell. Therefore, additional sacrificial scavengers, while beneficial, do not represent a "rate-limiting" step in the cellular defense cascade, as they are unable to prevent any subsequent effects once the radicals are no longer "free."

In addition, currently available radiation protection agents must be administered either before the radiation exposure, or immediately after radiation exposure, for example within 4 hours after the radiation exposure. Landauer et al., "Genistein treatment protects mice from ionizing radiation injury." J APPL TOXICOL 23(6):379-85 (2003); Vijay-Kumar et al., "Flagellin treatment protects against chemicals, bacteria, viruses, and radiation." J IMMUNOL 180(12):8280-5 (2008). Unfortunately, however, it may not be possible to treat for exposure to radiation within 4 hours of the exposure. Therefore, there is a significant need in the art for effective radiation protection agents, and in particular post-radiation protection agents that are effective when administered more than 24 hours after radiation exposure.

### SUMMARY OF THE INVENTION

The present disclosure is generally directed to methods of treating or managing the effects of exposure to radiation in a subject or organism, for example a mammal such as a human, well after the organism has been exposed to the radiation, for example more than 24 hours after the organism has been exposed to radiation. These methods involve the administration of a therapeutically effective amount of RLIP76 protein or an effective portion of RLIP76 protein to the organism after exposure to radiation, *i.e.,* post-radiation. Surprisingly, as disclosed herein, an organism continues to benefit from administration of the RLIP76 protein or effective portions thereof more than 24 hours after exposure to radiation. As used herein, "an effective portion of RLIP76 protein" or "effective portions thereof" or "active fragments" are any RLIP76 protein fragment(s), protein portion(s), or combinations thereof which promote the treatment of the effects of exposure to radiation in a cell or organism. The RLIP76 protein may be a recombinant RLIP76 protein or fragments or portions thereof. The methods disclosed herein do not include administration to the organism of the RLIP76 protein or effective portions thereof before or prior to exposure to radiation.

In certain embodiments, the RLIP76 protein or effective portions thereof is administered to the organism one or more times more than 24 hours after radiation exposure. The RLIP76 protein or effective portions thereof can be administered one or more times about 25 hours, 26 hours, 27 hours, 28 hours, 29 hours, 30 hours, 31 hours, 32 hours, 33 hours, 34 hours, 35 hours, 36 hours, 42 hours, 48 hours, 60 hours, 72 hours, 84 hours, 96 hours, 4.5 days, 5 days, 5.5 days, 6 days, 6.5 days, 7 days, 7.5 days, 8 days, 8.5 days, 9 days, 9.5 days, 10 days, 10.5 days, 11 days, 11.5 days, 12 days, 12.5 days, 13 days, 13.5 days, 2 weeks, 3 weeks, 4 weeks, 5 weeks, 6 weeks, 7 weeks, 8 weeks or longer after radiation exposure.

In other embodiments, the RLIP76 protein or effective portions thereof is administered to the organism two or more times after radiation exposure: first, at least once within 24 hours after radiation exposure, and second, at least once more than 24 hours after radiation exposure. The first dose of RLIP76 protein or effective portions thereof may be administered to the organism at about 1 hour, 2 hours, 3 hours, 4 hours, 5 hours, 6 hours, 7 hours, 8 hours, 9 hours, 10 hours, 11 hours, 12 hours, 13 hours, 14 hours, 15 hours, 16 hours, 17 hours, 18 hours, 19 hours, 20 hours, 21 hours, 22 hours, 23 hours, or 24 hours after radiation exposure. One or more such doses may be administered within 24 hours after radiation exposure. The second dose of RLIP76 protein or effective portions thereof may be administered to the organism at about 30 hours, 36 hours, 42 hours, 48 hours, 60 hours, 3 days, 3.5 days, 4 days, 4.5 days, 5 days, 5.5 days, 6 days, 6.5 days, 7 days, 7.5 days, 8 days, 8.5 days, 9 days, 9.5 days, 10 days, 10.5 days, 11 days, 11.5 days, 12 days, 12.5 days, 13 days, 13.5 days, 2 weeks, 3 weeks, 4 weeks, 5 weeks, 6 weeks, 7 weeks, 8 weeks or longer after radiation exposure. One or more such doses may be administered more than 24 hours after radiation exposure. The doses administered within 24 hours and more than 24 hours after radiation exposure may have approximately the same amount of RLIP76 protein or active fragments thereof, or may have different amounts. In addition, if two or more doses are given within each of these respective time frames, each dose may have approximately the same amount of RLIP76 protein or active fragments thereof, or may have different amounts.

In certain embodiments, the RLIP76 protein or active fragments thereof can be administered from one time to ten times after 24 hours post-radiation. In a further embodiment, the RLIP76 protein or active fragments thereof can be administered from one time to five times after 24 hours post-radiation. In a further embodiment, the RLIP76 protein or active fragments thereof can be administered from three times to ten times after 24 hours post-radiation. In a further embodiment, the RLIP76 protein or active fragments thereof can be administered from two times to eight times after 24 hours post-radiation.

In certain embodiments, the RLIP76 protein or active fragments thereof is administered at least three times after 24 hours post-radiation. In a further embodiments, the RLIP76 protein or active fragments thereof can be administered at least five times after 24 hours post-radiation.

In certain embodiments, the RLIP76 protein or active fragments thereof is administered one or more times within 24 hours post-radiation and/or one or more times after 24 hours post-radiation. In certain embodiments, the RLIP76 protein or active fragments thereof is administered once within 24 hours post-radiation and one or more times after 24 hours post-radiation. In certain embodiments, the RLIP76 protein or active fragments thereof is administered one or more times within 24 hours post-radiation and no further administration after 24 hours post-radiation. In certain embodiments, the RLIP76 protein or active fragments thereof can be administered one or more times between 25 hours and three months post-radiation. In a further embodiment, the RLIP76 protein or active fragments thereof can be administered one or more times between 25 hours and one month post-radiation. In a further embodiment, the RLIP76 protein or active fragments thereof can be administered one or more times between two months and three months post-radiation. In a further embodiment, the RLIP76 protein or active fragments thereof can be administered one or more times between 48 hours and two months post-radiation. In a further embodiment, the RLIP76 protein or active fragments thereof can be administered between one week and one month post-radiation. In a further embodiment, the RLIP76 protein or active fragments thereof is administered after 24 hours post-radiation for the purpose of extending phramacodynamics of previously administered RLIP76 protein or active fragments thereof. In a further embodiment, the RLIP76 protein or active fragments thereof is administered after 24 hours post-radiation for the purpose of extending a pharmaceutical effect of previously administered RLIP76 protein or active fragments thereof.

In certain embodiments, the RLIP76 protein or active fragments thereof is comprised within a liposome, which can also be referred to as a proteoliposome or proteoliposome composition. In some embodiments, the RLIP proteoliposomes are delivered in a pharmaceutically acceptable carrier.

Certain embodiments of the present disclosure are directed to methods of treating the effects of radiation exposure in an organism in need of such treatment, comprising administering an effective amount of RLIP76 protein or an effective portion thereof to the organism more than 24 hours after the radiation exposure. Other embodiments are directed to methods of treating the effects of exposure to radiation in an organism in need of such treatment, comprising administering (a) at least a first dose of an effective amount of RLIP76 protein or an effective portion thereof to the organism within 24 hours after the radiation exposure, and (b) at least a second dose of an effective amount of RLIP76 protein or an effective portion thereof to the organism more than 24 hours after radiation exposure. In some embodiments, the organism is a mammal, for example a human. The radiation that the organism is exposed to may be ionizing radiation, including but not limited to X-radiation, γ-radiation, energetic electron radiation, ultraviolet radiation, thermal radiation, cosmic radiation, electromagnetic radiation, nuclear radiation, or a combination thereof. In still other embodiments, the energetic electron radiation is β-particle radiation or the radiation is proton or heavy ion radiation.

In certain embodiments, the RLIP76 protein or an effective portion thereof is administered more than 24 hours after the exposure to the radiation. In other embodiments, the RLIP76 protein or an effective portion thereof is administered within 24 hours after the exposure to the radiation, for example at about the time of exposure to the radiation, and more than 24 hours after the exposure to the radiation. The RLIP76 protein or an effective portion thereof as disclosed herein may be administered in one or more doses to the mammal, in either or both relevant time periods, *i.e.,* within 24 hours after the exposure to the radiation and/or more than 24 hours after the exposure to the radiation. For example, any of these embodiments can be such that the RLIP76 protein is administered multiple times to the mammal in various combinations, including but not limited to more than 24 hours after the exposure to the radiation, or within 24 hours after the exposure to the radiation and more than 24 hours after the exposure to the radiation. In each of the relevant above embodiments, the doses may comprise about the same amount of the RLIP76 protein or an effective portion thereof, or may comprise different amounts of the RLIP76 protein or an effective portion thereof.

In certain embodiments, the RLIP76 protein or an effective portion thereof is administered to the organism more than 24 hours, more than 36 hours after, more than 48 hours, more than 60 hours, more than 72 hours, more than 84 hours, and/or more than 96 hours after the radiation exposure. For example, in certain embodiments the doses may be administered at +24 hours, +48 hours, +72 hours, and +96 hours after radiation exposure, respectively. In other embodiments, the doses may be administered at 48 hours and 96 hours after radiation exposure, at 24 hours and 72 hours after radiation exposure, at 0 hours, 48 hours, and 96 hours after radiation exposure, at 14 hours and 48 hours after radiation exposure, at 16 hours and 64 hours after radiation exposure, or at 1 hour, 24 hours, and 48 hours after radiation exposure.

The RLIP76 protein or an effective portion thereof may be administered to a mammal in need thereof as disclosed herein at a dosage of between about 0.5 mg/kg body weight and about 14.0 mg/kg body weight, for example about 1.0 mg/kg body weight, about 1.5 mg/kg body weight. about 2.0 mg/kg body weight, about 2.5 mg/kg body weight, about 3.0 mg/kg body weight, about 3.5 mg/kg body weight, about 4.0 mg/kg body weight, about 4.5 mg/kg body weight, about 5.0 mg/kg body weight, about 5.5 mg/kg body weight, about 6.0 mg/kg body weight, about 6.5 mg/kg body weight, about 7.0 mg/kg body weight, about 7.5 mg/kg body weight, about 8.0 mg/kg body weight, about 8.5 mg/kg body weight, about 9.0 mg/kg body weight, about 9.5 mg/kg body weight, about 10.0 mg/kg body weight, about 10.5 mg/kg body weight, about 11.0 mg/kg body weight, about 11.5 mg/kg body weight, about 12.0 mg/kg body weight, about 12.5 mg/kg body weight, about 13.0 mg/kg body weight, or about 13.5 mg/kg body weight.

In certain embodiments, the RLIP76 protein or active fragments thereof is administered at a dosage of at least 0.01 µg/kg body weight. In a further embodiment, the RLIP76 protein or active fragments thereof is administered at a dosage of at least 0.1 µg/kg body weight. In a further embodiment, the RLIP76 protein or active fragments thereof is administered at a dosage of at least 1 µg/kg body weight. In a further embodiment, the RLIP76 protein or active fragments thereof is administered at a dosage of at least 5 µg/kg body weight. In a further embodiment, the RLIP76 protein or active fragments thereof is administered at a dosage of at least 0.1 mg/kg body weight.

In certain embodiments, the RLIP76 protein or active fragments thereof is administered at a dosage of between about 0.01 µg/kg body weight and about 100 mg/kg body weight. In a further embodiment, the RLIP76 protein is administered at a dosage of between about 0.1 µg/kg body weight and about 50 mg/kg body weight. In a further embodiment, the RLIP76 protein is administered at a dosage of between about 1 µg/kg body weight and about 40 mg/kg body weight. In a further embodiment, the RLIP76 protein is administered at a dosage of between about 5 µg/kg body weight and about 25 mg/kg body weight. In a further embodiment, the RLIP76 protein is administered at a dosage of between about 0.1 mg/kg body weight and about 10 mg/kg body weight.

In certain embodiments, the RLIP76 protein or active fragments thereof is administered via an administration route selected from the group consisting of intravenous, intramuscular, subcutaneous, intraperitoneal, and oral administration. In a further embodiment, the RLIP76 protein is administered via an administration route selected from the group consisting of intravenous (iv), subcutaneous (sc), and oral administration (po).

In certain embodiments, the radiation exposure or the exposure to radiation is least 2 Gy or 200 cGy. In a further embodiment, the radiation exposure or the exposure to radiation is at least 5 Gy or 500 cGy. In a further embodiment, the radiation exposure or the exposure to radiation is at least 7.5 Gy or 750 cGy. In a further embodiment, the radiation exposure or the exposure to radiation is least 10 Gy or 1000 cGy. In a further embodiment, the radiation exposure or the exposure to radiation is at least 20 Gy or 2000 cGy. In a further embodiment, the radiation exposure or the exposure to radiation is between about 200 cGy and about 5000 cGy. In a further embodiment, the radiation exposure or the exposure to radiation is between about 2 Gy and about 100 Gy. In a further embodiment, the radiation exposure or the exposure to radiation is between about 3 Gy and about 50 Gy.

In certain embodiments, the radiation exposure or the exposure to radiation is an extended radiation exposure of at least 0.2 Gy. In certain embodiments, the radiation exposure or the exposure to radiation is an extended radiation exposure of at least 0.5 Gy. In certain embodiments, the radiation exposure or the exposure to radiation is an extended radiation exposure of between about 0.5 Gy and about 2.0 Gy. In a further embodiment, the extended radiation exposure is at least 2 hours. In a further embodiment, the extended radiation exposure is at least 12 hours. In a further embodiment, the extended radiation exposure is between about 2 hours to 48 hours.

In certain embodiments, pharmaceutical effects of the administered RLIP76 protein or an effective portion thereof are monitored using biomarkers of radiation damage. In a further embodiment, the biomarkers of radiation damage include DNA abnormalities. In a further embodiment, the biomarkers of radiation damage include microsatellite bodies in peripheral reticulocytes. In a further embodiment, the biomarkers of radiation damage include DNA abnormalities or microsatellite bodies in peripheral reticulocytes. In a further embodiment, the biomarkers of radiation damage include DNA abnormalities and microsatellite bodies in peripheral reticulocytes. In other embodiments, pharmaceutical effects of the administered RLIP76 protein or an effective portion thereof are monitored using death rate of tested subject.

In any of the above embodiments, a second radiation protection agent may be administered to the organism, either concurrently with, or in combination with, the RLIP76 protein or an effective portion thereof. In certain embodiments, the second radiation protection agent may be a free radical scavenger, an antioxidant, or a superoxide dismutase analog. The RLIP76 protein or an effective portion thereof as disclosed herein may be administered in a pharmaceutical composition or proteoliposomal composition. In other embodiments, the pharmaceutical composition or proteoliposomal composition further comprises a lectin, a glycolipid, a phospholipid, or a combination thereof. In other embodiments, the RLIP76 protein or an effective portion thereof is a recombinant protein or a portion thereof. The pharmaceutical composition or proteoliposomal composition of the present disclosure may be administered subcutaneously, intravenously, topically, orally, non-orally, or a combination thereof.

Throughout this disclosure, unless the context dictates otherwise, the word "comprise" or variations such as "comprises" or "comprising" is understood to mean "includes, but is not limited to" such that other elements that are not explicitly mentioned may also be included. Further, unless the context dictates otherwise, use of the term "a" may mean a singular object or element, or it may mean a plurality, or one or more of such objects or elements. In addition, the use of "or" herein means "and/or" unless specifically stated otherwise. Also, terms such as "element" or "component" encompass both elements and components comprising one unit and elements or components that comprise more than one unit unless specifically stated otherwise.

It is to be understood that both the foregoing general description and the following detailed description are exemplary and explanatory only, and are not restrictive of the invention, as claimed. The section headings used herein are for organizational purposes only and are not to be construed as limiting the subject matter described. All documents, or portions of documents, cited in this application, including, but not limited to, patents, patent applications, articles, books, and treatises, are hereby expressly incorporated herein by reference in their entirety for any purpose. In the event that one or more of the incorporated literature and similar materials defines a term in a manner that contradicts the definition of that term in this application, this application controls.

### BRIEF DESCRIPTION OF THE DRAWINGS

The following drawings form part of the present specification and are included to further demonstrate certain aspects of the present invention. The present invention may be better understood by reference to one or more of these drawings in combination with the detailed description of specific embodiments presented herein.
**Figure 1****.** Mechanisms of cell death by different toxic insults.
**Figure 2****.** Examples of the physiological significance of RLIP76.
**Figure 3A** and **Figure 3B****.** Baseline survival curves of mice treated with varying dosages of X-irradiation. **Figure 3A****.** Survival at different radiation doses in control treated mice. **Figure 3B****.** Mean time to death versus radiation dose.
**Figure 4****.** The effect of RLIP76 on radiation sensitivity. The three groups shown are: without treatment with liposomes (circle); treatment with liposomes without RLIP76 (square); and treatment with liposomes with RLIP76 (triangles).
**Figure 5****.** Effect of RLIP76 on survival after higher doses of X irradiation. Four wild-type (+/+) RIP1 mice and four homozygous (-/-) mice were each exposed to 750 cGy X irradiation and treated with control liposomes administered by i.p. injection (triangles, homozygous RIP1 mice; diamonds, wild-type RIP mice), or 400 µg RLIP76 liposomes administered by i.p. injection (squares, homozygous RIP1 mice; circles, wild-type RIP1 mice), given 12 hours after exposure.
**Figure 6****.** Dose response of RLIP76 on survival after 500 cGy exposure: diamonds, control (untreated) mice; squares, mice treated with 25 µg of RLIP76 liposomes 14 hours and 48 hours after exposure; triangles, mice treated with 50 µg of RLIP76 liposomes 14 hours and 48 hours after exposure; X's, mice treated with 100 µg of RLIP76 liposomes 14 hours and 48 hours after exposure; stars, mice treated with 100 µg of RLIP76 liposomes 24 hours and 72 hours after exposure; circles, mice treated with 100 µg of RLIP76 liposomes 48 hours and 96 hours after exposure.
**Figure 7****.** Dose-reduction factor graph measuring 50% survival versus radiation dose of untreated and RLIP76 treated mice.
**Figure 8****.** Effect of time of administration of RLIP76 on survival after 500 cGy exposure: diamonds, control (untreated) mice; squares, mice treated with 25 µg of RLIP76 liposomes 14 hours and 48 hours after exposure; triangles, mice treated with 25 µg of RLIP76 liposomes 0 hours, 48 hours, and 96 hours after exposure.
**Figure 9****.** Effect of RLIP76 at higher radiation doses: diamonds, 750 cGy control (untreated); squares, 750 cGy treated with 100 µg of RLIP76 liposomes 0 hours, 48 hours, and 96 hours after exposure; triangles, 1000 cGy control (untreated); X's, 1000 cGy treated with 100 µg of RLIP76 liposomes 16 hours and 64 hours after exposure.

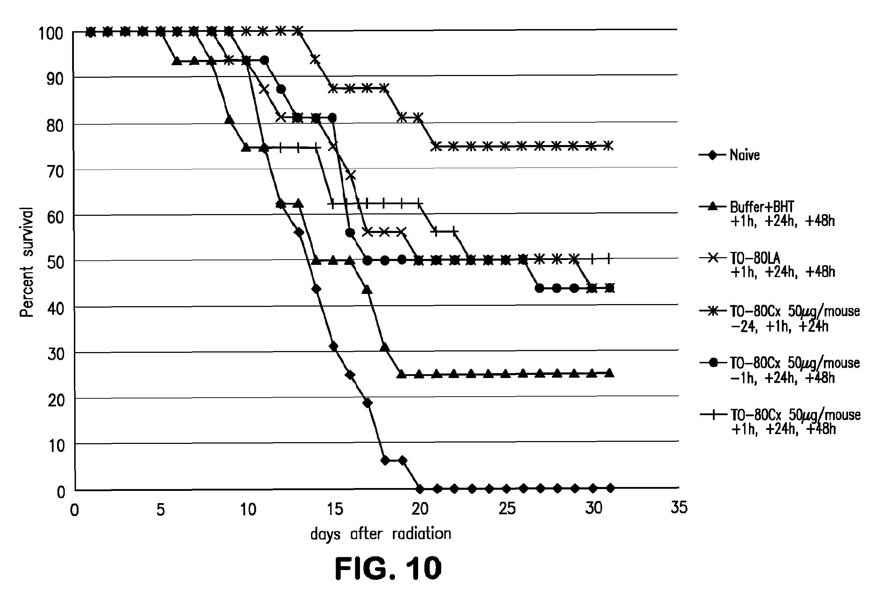

**Figure 10** shows overall survival rate of mice after gamma irradiation. Fourteen-week old CD2FI male mice were grouped into individual cohorts of 16 mice each and exposed to 9.25 Gy whole body gamma radiation delivered at 0.6 Gy/minute via cobalt60 source. The mice were treated with multiple doses of various formulations of liposomes, RLIP76 protein and antioxidant (BHT) via i.p. injection as shown. A complex of RLIP76 protein, liposomes, and the antioxidant BHT is designated TO-80Cx (the 80 refers to the mean size of the liposomes of 80 nm, which classifies them as intermediate sized vesicles); TO-80LA refers to the liposomes constituted in buffer with BHT but without RLIP76 protein. Times are in reference to hours before or after radiation exposure and the dose of 50 µg is the amount of RLIP76 protein contained in the total volume of TO-80Cx delivered with each dose. This amount represents a dose of 1.67 mg RLIP76 protein per kilogram of body weight of each mouse. The x-axis is the measure in days after radiation exposure and the y-axis is the percentage of each cohort alive on that day.
**Figure 11** is a different depiction of the percentage of mice from each cohort still alive at 30 days after gamma irradiation.
**Figure 12** shows the overall survival of additional cohorts of CD2F1 mice treated under the same conditions as described, where the treatments are various delivery vehicles and controls delivered at the times specified after exposure. In the legend, 5 AED refers to 5-andrestenediol.

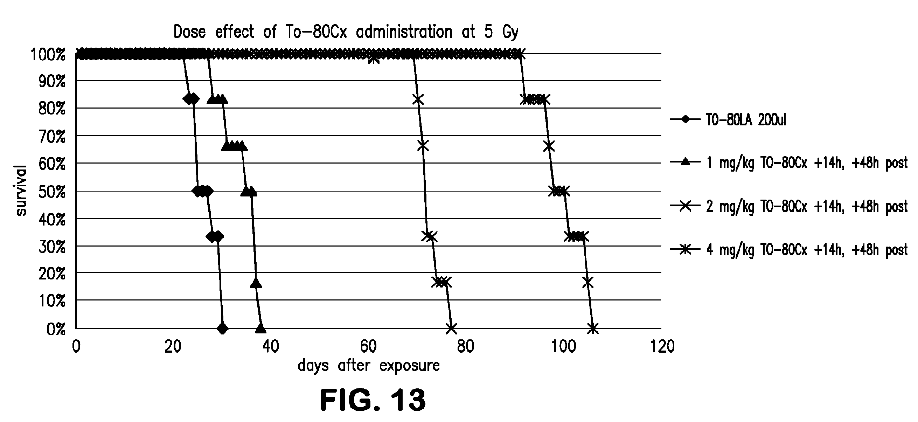

**Figure 13** shows overall survival rate of C57/B16 mice after a lower exposure to gamma irradiation, 5 Gy total body exposure, delivered via 6-MeV photon beam at a rate of 4 Gy/minute. In this experiment, the mice were treated with two doses of TO-80Cx administered 14 and 48 hours after exposure delivered via oral gavage. Dose levels shown are amount of RLIP76 protein contained in the volume of TO-80Cx delivered and are expressed as amount of protein per total body weight. The x-axis is the measure in days after radiation exposure and the y-axis is the percentage of each cohort alive on that day.

### DETAILED DESCRIPTION OF THE INVENTION

The present disclosure arises at least in part from the discovery by the inventor that RLIP76 protein and effective portions (e.g., active fragment) thereof (also referred to herein as RLIP76) are surprisingly effective in protecting organisms against the toxic effects of radiation exposure, even when administered significantly after the radiation exposure. Thus, unlike other radiation protection candidates, RLIP76 continues to protect an organism against the toxic effects of radiation exposure more than 24 hours after radiation exposure. While use of RLIP76 to treat radiation exposure has been disclosed in U.S. Serial No. 11/741,447, U.S. Publ. No. 20080279919, which is incorporated herein by reference, the ability of RLIP76 to continue to offer therapeutic protection or benefit to an organism significantly after radiation exposure is both surprising and an important benefit, given the logistical difficulties of treating large numbers of humans after an event that results in significant radiation exposure. Another significant benefit of RLIP76 is that it may be administered orally, for example using RLIP76 proteoliposome compositions, as supported by preliminary animal studies. Nevertheless, it is understood that delivery may also be accomplished by other routes of administration known in the art, including, but not limited to, sublingual, buccal, intraperitoneal, inhalation, intravenous, intramuscular, transmucosal, or transdermal delivery. In addition to delivery of RLIP76, the RLIP76 proteoliposomes disclosed herein are contemplated to be effective for delivery of other radiation protection agents to the organism.

### RLIP76

RLIP76 (also known as RALBP1 or RIP1) is a ubiquitous protein found in *Drosophila* to humans that serves multiple roles in cellular physiology. When membrane-associated, the protein functions as a multi-specific efflux pump for a variety of compounds, including amphiphilic small molecules such as *Vinca* alkaloids and anthracylines, which are common anticancer drugs. However, RLIP76 transport also involves movement from the cell of endogenous glutathione electrophile conjugates (GS E) formed from reactive oxygen species (ROS). ROS are produced by a variety of insults such as radiation and a plethora of organic chemicals, and are toxic to the cell on many levels. As their name implies, ROS are highly reactive and bind to almost anything in their path, including proteins, lipids and nucleic acids, modifying each of these as they are contacted. The damage done by ROS to lipids (lipid peroxidation) is particularly pernicious since the resulting peroxidation products are themselves toxic. These include proapoptotic reactive alkenals, such as 4 hydroxynonenal (4-HNE), which are long lived and can accumulate in the cell, ultimately leading to further damage and death. As such, RLIP76 is an important component of stress response in cultured cells and provides protection from stressors including heat, oxidant chemicals, chemotherapeutic agents, UV irradiation and X-irradiation.

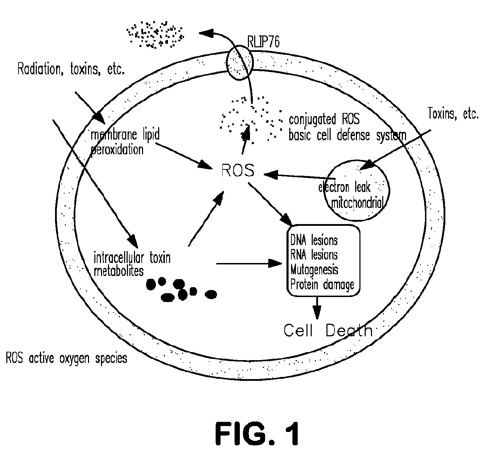

The normal cell has defense mechanisms designed to bind up (conjugate) these ROS-associated toxins, chief of which is glutathione. Glutathione binds electrophilic compounds to sequester the reactive electrons. However, the resulting conjugates (GS-E) are harmful or fatal to the cell if allowed to accumulate, and so must be removed by the cell. Although not wishing to be bound to any particular theory, it appears that the active efflux of GS-E derived from these toxic intermediates is the principal mechanism by which RLIP76 confers resistance to oxidant and radiant stressors **(****Figure 1****).**

The protective effect of RLIP76 goes beyond its protection of potentially toxic chemical substituents and their by-products. For example, electrophilic products of lipid peroxidase (LPO) caused by reactive oxygen species generated during radiation may partly account for cell killings by radiation. As detailed herein, RLIP76-mediated transport of GSH conjugates of these electrophiles provides protection from radiation. Such protection may be readily transferred to a larger scale to protect mammals against damaging radiation, including ionizing, electromagnetic, thermal, and laser radiation, wherein either long-or short-range electrons are involved.

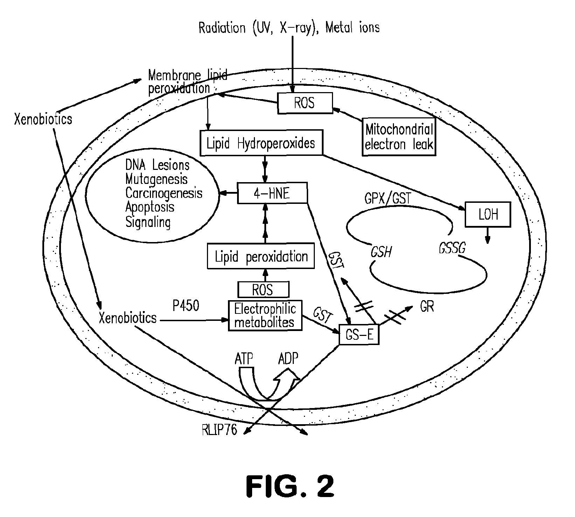

Therefore, RLIP76 mediates transport of endogenously generated chemicals, metabolic products, their by-products and exogenously administered drugs or radiation, and their by-products. RLIP76 mediates the transport of most chemicals and by-products that also involve GS-E (*e*.*g*., conjugate of 4-HNE). For example, RLIP76-enriched cells are resistant to toxicity in the form of chemical toxicity (organic or inorganic) or from damage (*e.g.,* from stress, oxidation, alkylation, radiation). The function of RLIP76 via an ATP-dependent efflux of xenobiotics (*e.g*., GS-E and exogenous and endogenous electrophiles) is shown in **Figure 2****.** Here, xenobiotics, radiation, their metabolites, mitochondrial electron transport and metal ions generate ROS that can cause membrane lipid peroxidation and 4-hydroxynonenal (the toxic end product of lipid peroxidation), which can cause DNA damage leading to mutagenesis, carcinogenesis and apoptosis as well as modulate the stress mediated signaling pathways. RLIP76 mediates the ATP-dependent efflux of a wide variety of metabolic, stress, and pharmaceutical by-products, such as amphiphilic drugs, GSH-conjugates (GS-E) of both xeno and endo-biotics, GS-HNE and leukotrienes, from eukaryotic cells. The transport of GS-E is important for maintaining functionality of GSTs and glutathione reductase (GR), because these enzymes are inhibited by GS-E. RLIP76 regulates the intracellular concentrations of 4-HNE by a coordinated mechanism with cellular GSTs.

### Structure of RLIP76

The primary structure of RLIP76 reveals several interesting features. The protein may be divided into four regions out of which two central domains carry a Rac1/CDC42 GAP activity and a Ral binding domain. The function of the two flanking domains is still unknown. Representative nucleotide sequences of human RLIP76 (GenBank Accession Number NM_006788) and mouse RLIP76 (NM_009067), and amino acid sequences of human RLIP76 (GenBank Accession Number NP_006779) and mouse RLIP76 (GenBank Accession Number NP_033093), have been described. The human RLIP76 amino acid sequence includes sites for N-glycosylation (amino acids 341-344), cAMP (amino acids 113-116), cGMP-dependent protein kinase phosphorylation (amino acids 650 653), tyrosine kinase phosphorylation (amino acids 308-315), N-myristolation (amino acids 21-26, 40-45, and 191-196), leucine zipper pattern (amino acids 547-578) and several protein kinase C phosphorylation, casein kinase II phosphorylation, trypsin and chymotrypsin cut sites. The presence of such motifs in the primary structure of RLIP76, and its facile proteolytic degradation, shows RLIP76 to be involved in several intra- and extracellular processes (*e.g*., protein processing, intracellular signaling, protein degradation, recognition, tagging, *etc.*) and that proteolytic processing of RLIP76 is required for the multiple functions. The peptide fragments of RLIP76 individually or in association with other fragments may catalyze these various functions. For example, N terminal and C-terminal fragments of RLIP76, fragments that are individually incapable of mediating ATP-dependent transport, can catalyze the transport of electrically charged drugs (*e.g.*, DOX, colchicines) when reconstituted together in proteoliposomes.

In some embodiments, the RLIP76 protein of the invention comprises a sequence of 655 amino acids as set forth in GenBank Accession Number NP_006779). In some embodiments, the RLIP76 protein of the invention comprises a sequence as disclosed in US 2005/0123594, US 2006/0182749, US 2008/0279919, US 2010/0124566, or WO 2009/100446A1, the contents of which are incorporated by reference in their entireties.

Unlike the ABC transporters, no transmembrane alpha-helices are evident in the RLIP76 sequence. The association of RLIP76 with membranes has, however, been demonstrated by immunohistochemical studies using specific antibodies (Awasthi, et al., Proceedings of the American Association for Cancer Research, 43:Abst. 4717, 2002; herein incorporated by reference). The extraction of RLIP76 from cell lysates requires detergent, suggesting membrane association, a feature important for transport. These findings show a greater diversity in this transporter, in terms of structural elements defining ATP binding and mode of membrane insertion, than is currently accepted. In addition, the distinction between transporters for anions as opposed to neutral or cationic substrates is blunted because RLIP76 catalyzes the transport of both, and, in contrast to MRP 1, does so without co-transporting GSH.

RLIP76 expressed in cultured cells or in *E. coli* undergoes facile proteolysis during purification. The most prominent peptides, N-RLIP761-367 and C-RLIP76410-655, arising from the N and C termini of RLIP76, respectively, appear as 49 kDa and 38 kDa bands in SDS-gels. Both these peptides display constitutive ATPase activity that may be stimulated in the presence of the anionic or cationic ligands transported by RLIP76. Both peptides bind ATP, as shown by photoaffinity labeling that increased in the presence of vanadate, indicating the trapping of a reaction intermediate in the ATP binding site. Neither of the two fragments catalyze transport when reconstituted alone in proteoliposomes. However, when reconstituted together, ATP dependent transport of charged chemicals (*e.g.*, DNP-SG, DOX) is observed with kinetic parameters similar to those for RLIP76. The ATP binding sites in N-RLIP761-367 and C RLIP76410-655 were identified to be amino acids 69-74 and amino acids 418-425, respectively. Mutations of K74 and K425 in the N and C-terminal peptides, respectively, abrogate the ATPase activity, ATP binding capacity, and transport function. The sequence of these ATP binding sites is not identical to the consensus sequence for the P-loop (Walker motif).

In addition to the human RLIP76 nucleic acid sequence described above, a number of single nucleotide polymorphisms (SNPs) have been described in the art within the human RLIP76 gene, three of which (an A to G mutation at nucleotide 660 of the coding sequence, a G to A mutation at nucleotide 838 of the coding sequence, and a C to T mutation at nucleotide 2065 of the coding sequence) fall within the RLIP76 coding sequence. These nucleotide changes result in changing the amino acid sequence from lysine to glutamate at amino acid position 149, from arginine to glutamine at amino acid position 208, and from alanine to valine at amino acid position 617, respectively. These SNPs, along with SNPs that occur in the introns of the human RLIP76 gene, and well as SNPs that occur in the 5' and 3' untranslated regions of the human RLIP76 gene, are described in the Single Nucleotide Polymorphism (SNP) database on the National Center for Biotechnology Information web site.

In certain aspects of the present disclosure, "RLIP76 protein" can refer to the full length human RLIP76 amino acid sequence as shown in GenBank Accession Number NP_006779, one or more fragments of human RLIP76 amino acid sequence that alone or in combination retain RLIP76 transport activity, or mutations of the human RLIP76 amino acid sequence that retain RLIP76 transport activity. In certain embodiments, RLIP76 can refer to an amino acid sequence that has about 99% identity or homology with the human RLIP76 amino acid sequence as shown in GenBank Accession Number NP_006779, about 98% identity or homology, about 95% identity or homology, about 90% identity or homology, about 85% identity or homology, or about 80% identity or homology to the human RLIP76 amino acid sequence as shown in GenBank Accession Number NP_006779. The percentage of sequence identity or homology may reflect certain additions, deletions, substitutions, silent or conservative mutations to the sequences.

### Liposomes

Liposomes are vesicles consisting of amphipathic lipids arranged in one or more concentric bilayers. When lipids are placed in aqueous medium, the hydrophilic interaction of the lipid head groups with water results in the formation of multilamellar and unilamellar systems or vesicles which resemble biological membranes in the form of a spherical shell. Liposomes may be small (0.025-0.05 µm) to large (0.05-10 µm) multilamellar vesicles. Lipids used to prepare the liposomes can include, but are not limited to, phospholipids, sphingolipids, glycosphingolipids, saturated glycerides, steroids (*e.g*., cholesterol) and synthetic phospholipids. Liposomes are typically prepared by melting the lipid together in aqueous solvent with an emulsifier like POE. The agent is then added and the liposomes are generated through mixing or sonication. The agent is usually entrapped in the vesicle structure. These basic liposomes are sometimes referred to as "conventional liposomes." Several other types of liposomal preparations exist, including (1) sterically stabilized liposomes, which are surface coated with an inert hydrophilic polymer, such as polyethylene glycol; (2) targeted liposomes, to which are attached targeting ligands, such as antibodies or fragments thereof, lectins, oligosaccharides or peptides (*e.g*., choleratoxin B (CTB) is used to target liposomes to the gastrointestinal epithelium); and (3) reactive or "polymorphic" liposomes, which change their phase and structure in response to a particular interaction (this group includes liposomes sensitive to ions (pH, cations), heat and light, among other stimuli).

In certain embodiments the compositions include proteoliposomes. As used herein, a "proteoliposome" is generally a protein and lectin or glyco- or phospholipid combination that forms a spherical micellular-like or vesicular structure. The structures may form spontaneously or by chemical or mechanical manipulation, or combinations thereof. Proteoliposomes take advantage of the amphipathic nature of the lipid (or lectin) that causes them to form bilayers when in solution resulting in at least one of several shapes, including: (a) spherical micelle with the tails inward, or (b) bimolecular sheets that are bilayers with hydrophobic tails sandwiched between hydrophilic head groups. In general, proteoliposomes may reseal themselves when torn or broken. Proteoliposomes may contain only one lectin or lipid or a variety and combination of each. Examples of phospholipids include phosphatidylcholine, sphingomyelin, phosphatidylserine, inositol phospholipids, and phosphatidylethanolamine. When used, proteoliposomes may be charged or electrically neutral and are generally used at physiological pH. They may also be structures mixed with detergent (*e.g.,* detergent/lipid/protein, detergent/lectin/protein). Methods for preparing proteoliposomes of defined lipid-protein or lectin-protein ratios and size are well-known to one of ordinary skill in the art of molecular biology and protein/lipid biochemistry. The proteoliposomes of the disclosure can be made by any method known in the art, including methods disclosed and described in U.S. Patent Application Serial No. 10/713,578, published as US 2005/0123594 A1, the disclosure of which is incorporated herein in its entirety by reference for all purposes.

### Additional Radiation Protection Agents

In certain aspects of the present disclosure, the compositions comprising RLIP76, for example the RLIP76 proteoliposomes, can be used in combination with one or more additional radiation protection agents, including, but not limited to, free radical scavengers, antioxidants, and superoxide dismutase analogs. Unprotected RLIP76 is susceptible to proteolysis, rendering administration of the bare protein challenging. To facilitate stability of the protein, RLIP76 may be administered in the form of lipid encapsulated proteoliposomes. In addition, RLIP76 protein may be administered along with one or more radiation protection agents, for example antioxidants, free radical scavengers, or superoxide dismutase analogs, to facilitate stability of the protein.

Additional free radical scavengers or antioxidants that can be used in combination with RLIP76 include, but are not limited to, butylated hydroxytoluene (BTH), N-acetylcysteine, sodium thiosulfate, glutathione ethyl ester, glutathione, D-methionine, cysteamine, cystamine, aminopropylmethylisothiourea, Ethyol, vitamin E, edaravone (3-methyl-1-phenyl-2-pyrazolin-5-one), melatonin, polynitroxyl-albumin, idebenone, nitric oxide, Carvedilol, alpha-lipoic acid, allopurinol, 2 O octadecylascorbic acid, N-2-mercaptopropionyl glycine, superoxide dismutase (SOD), recombinant human CuZn-SOD, glutathione peroxidase, catalase, nitric oxide synthase, ascorbic acid (Vitamin C), selenium, acetylcysteine, seleginine (Deprenyl®), pycnogenol, co-enzyme Q10, beta carotene, PC 01, SC-55858, iron (III) porphyrins, mithramycin, chromomycin, daunomycin, olivomycin and WP-631, or combinations thereof.

Additional radiation protection agents that can be used in combination with RLIP76 include, but are not limited to, Fullerene DF-1, butylated hydroxyanisole (BHA), butylated hydroxytoluene (BHT), carbon nanotubes, autologous and allogeneic bone marrow derived stem cells, CD34 positive cells, protein and/or cDNA and/or mRNA for Rad51 or Rad52 and related genes, TGF beta type II receptor gene and/or products, and p53 gene and/or products, or combinations thereof.

### Pharmaceutical Compositions and Routes ofAdministration

Therapeutic compositions comprising RLIP76 are provided herein as pharmaceutical preparations for systemic, topical or local administration to patients or subjects. The term "patient" or "subject" as used herein refers to human or animal subjects (animals being particularly useful as models for clinical efficacy of a particular composition). Selection of a suitable pharmaceutical preparation depends upon the method of administration chosen, and may be made according to protocols well known to medicinal chemists.

In certain embodiments, the compositions disclosed herein also comprise a pharmaceutically acceptable carrier. As used herein, "pharmaceutically acceptable carrier" includes any and all solvents, dispersion media, coatings, antibacterial and antifungal agents, isotonic and absorption delaying agents and the like. The use of such pharmaceutically acceptable carriers with pharmaceutical active agents is well known in the art. Except insofar as any conventional media or agent is incompatible with the active agent, its use in the compositions disclosed herein is contemplated. Supplementary active ingredients can also be incorporated into the compositions.

As used herein, "pharmaceutically-acceptable salts" refer to derivatives of RLIP76 or other compounds disclosed herein wherein the parent compound is modified by making acid or base salts thereof. Examples of pharmaceutically-acceptable salts include, but are not limited to, mineral or organic acid salts of basic residues such as amines; alkali or organic salts of acidic residues such as carboxylic acids; and the like. Thus, the term "acid addition salt" refers to the corresponding salt derivative of a parent compound that has been prepared by the addition of an acid. The pharmaceutically-acceptable salts include, but are not limited to, the conventional salts or the quaternary ammonium salts of the parent compound formed, for example, from inorganic or organic acids. For example, such conventional salts include, but are not limited to, those derived from inorganic acids such as hydrochloric, hydrobromic, sulfuric, sulfamic, phosphoric, nitric and the like; and the salts prepared from organic acids such as acetic, propionic, succinic, glycolic, stearic, lactic, malic, tartaric, citric, ascorbic, palmoic, maleic, hydroxymaleic, phenylacetic, glutamic, benzoic, salicylic, sulfanilic, 2-acetoxybenzoic, fumaric, toluenesulfonic, methanesulfonic, ethane disulfonic, oxalic, isethionic, and the like. Certain acidic or basic compounds may exist as zwitterions. All forms of the active agents, including free acid, free base, and zwitterions, are contemplated to be within the scope of the present disclosure.

A protein can be formulated into a composition in a neutral or salt form. Pharmaceutically acceptable salts include, but are not limited to, the acid addition salts (formed with the free amino groups of the protein) and which are formed with inorganic acids such as, for example, hydrochloric or phosphoric acids, or such organic acids as acetic, oxalic, tartaric, mandelic, and the like. Salts formed with the free carboxyl groups can also be derived from inorganic bases such as, for example, sodium, potassium, ammonium, calcium, or ferric hydroxides, and such organic bases as isopropylamine, trimethylamine, histidine, procaine and the like.

RLIP76 compositions can be complexed with polyethylene glycol (PEG), metal ions, or incorporated into polymeric compounds such as polylactic acid, polyglycolic acid, hydrogels, dextran, *etc.,* or incorporated into liposomes, microemulsions, micelles, unilamellar or multilamellar vesicles, erythrocyte ghosts or spheroblasts. Such compositions will influence the physical state, solubility, stability, rate of *in vivo* release, and/or rate of *in vivo* clearance, and are thus chosen according to the intended application.

In addition, RLIP76, or one or more active fragments thereof, can be bound, for example by covalent, non-covalent, ionic, or hydrophobic bonds, with any number of different delivery vehicles, including, but not limited to, liposomes, proteoliposomes, vesicles, nanoparticles, noisosomes, carrier proteins, gold particles, chitin, polymers, organic "cages," viruses, and bacteria. In addition, preferential uptake of any of the above RLIP76 compositions by one or more specific organs, tissues, or cell types can be accomplished by the inclusion of one or more specific targeting moieties with RLIP76 or any of the delivery vehicles listed above. Such targeting moieties include, but are not limited to, antibodies, or fragments thereof, peptides, lipids, chemicals, charged particles, receptors, proteins, viral promoters, transcription factors, DNA promoters, and nucleic acids that have a particular two- or three-dimensional structure.

The disclosed compounds can be orally administered, for example, with an inert diluent or with an assimilable edible carrier, or they can be enclosed in hard or soft shell gelatin capsule, or they can be incorporated directly with the food of the diet. For oral therapeutic administration, the active compounds can be incorporated with excipients and used in the form of tablets, troches, capsules, elixirs, suspensions, syrups, wafers, and the like. Such compositions and preparations should contain at least 0.1 % of active agent. The percentage of the compositions and preparations may, of course, be varied and may conveniently be between about 2 to about 60% of the weight of the unit. The amount of active agents in such therapeutically useful compositions is such that a suitable dosage will be obtained.

The tablets, troches, pills, capsules and the like may also contain the following: a binder, as gum tragacanth, acacia, cornstarch, or gelatin; excipients, such as dicalcium phosphate; a disintegrating agent, such as corn starch, potato starch, alginic acid and the like; a lubricant, such as magnesium stearate; and a sweetening agent, such as sucrose, lactose or saccharin may be added or a flavoring agent, such as peppermint, oil of wintergreen, or cherry flavoring. When the composition is a capsule, it may contain, in addition to materials of the above type, a liquid carrier. Various other materials may be present as coatings or to otherwise modify the physical form of the composition. For instance, tablets, pills, or capsules may be coated with shellac, sugar or both. A syrup of elixir may contain sucrose as a sweetening agent methyl and propylparabens as preservatives, a dye and flavoring, such as cherry or orange flavor. Of course, any material used in preparing any composition should be pharmaceutically pure and substantially non-toxic in the amounts employed. In addition, the active agents may be incorporated into sustained-release preparation and formulations.

The active agents may also be administered parenterally or intraperitoneally. Solutions of the active agents as free base or pharmacologically acceptable salts can be prepared in water suitably mixed with a surfactant, such as hydroxypropylcellulose. Dispersions can also be prepared in glycerol, liquid polyethylene glycols, and mixtures thereof and in oils. Under ordinary conditions of storage and use, these preparations contain a preservative to prevent the growth of microorganisms.

For parenteral administration in an aqueous solution, for example, the solution should be suitably buffered if necessary and the liquid diluent first rendered isotonic with sufficient saline or glucose. These particular aqueous solutions are especially suitable for intravenous, intramuscular, subcutaneous and intraperitoneal administration. In this connection, sterile aqueous media which can be employed will be known to those of skill in the art in light of the present disclosure. For example, one dosage could be dissolved in 1 mL of isotonic NaCl solution and either added to 1000 mL of hypodermoclysis fluid or injected at the proposed site of infusion, (see, for example, "Remington's Pharmaceutical Sciences" 15th Edition, pages 1035 1038 and 1570-1580). Some variation in dosage will necessarily occur depending on the condition of the subject being treated. The person responsible for administration will, in any event, determine the appropriate dose for the individual subject.

The pharmaceutical forms suitable for injectable use include sterile aqueous solutions or dispersions and sterile powders for the extemporaneous preparation of sterile injectable solutions or dispersions. In all cases the form must be sterile and must be suitably fluid. It must be stable under the conditions of manufacture and storage and must be preserved against the contaminating action of microorganisms, such as bacteria and fungi. The carrier can be a solvent or dispersion medium containing, for example, water, ethanol, polyol (for example, glycerol, propylene glycol, and liquid polyethylene glycol, and the like), suitable mixtures thereof, and vegetable oils. The proper fluidity can be maintained, for example, by the use of a coating, such as lecithin, by the maintenance of the required particle size in the case of dispersion and by the use of surfactants. The prevention of the action of microorganisms can be brought about by various antibacterial and antifungal agents, for example, parabens, chlorobutanol, phenol, sorbic acid, thimerosal, and the like. In many cases, it will be preferable to include isotonic agents, for example, sugars or sodium chloride. Prolonged absorption of the injectable compositions can be brought about by the use in the compositions of agents delaying absorption, for example, aluminum monostearate and gelatin.

Sterile injectable solutions are prepared by incorporating the active agents in the required amount in the appropriate solvent with several of the other ingredients enumerated above, as required, followed by filtered sterilization. Generally, dispersions are prepared by incorporating the various sterilized active agents into a sterile vehicle which contains the basic dispersion medium and the required other ingredients from those enumerated above. In the case of sterile powders for the preparation of sterile injectable solutions, the preferred methods of preparation are vacuum-drying and freeze-drying techniques which yield a powder of the active ingredient plus any additional desired ingredient from a previously sterile-filtered solution thereof.

In certain embodiments the disclosed compositions can be formulated to be administered by use of a skin patch, or transdermal delivery system. Transdermal administration can be accomplished by any of a number of systems known in the art. Examples of systems that may be adapted for use with the compositions described herein include those systems of transdermal administration described in U.S. Patent No. 4,816,252; U.S. Patent No. 5,122,382; U.S. Patent No. 5,198,223; U.S. Patent No. 5,023,084; U.S. Patent No. 4,906,169; U.S. Patent No. 5,145,682; U.S. Patent No. 4,624,665; U.S. Patent No. 4,687,481; U.S. Patent No. 4,834,978; and U.S. Patent No. 4,810,499, each of which is incorporated herein by reference.

These methods typically include an adhesive matrix or drug reservoir system and may include a skin permeation enhancement agent such as ethanol, polyethylene glycol 200 dilaurate, isopropyl myristate, glycerol trioleate, linolenic acid saturated ethanol, glycerol monooleate, glycerol monolaurate, n-decyl alcohol, capric acid, and certain saturated and unsaturated fatty acids, and their esters, alcohols, monoglycerides, acetate, diethanolamides and N,N-dimethylamides (see, for examples, U.S. Patent No. 4,906,169).

### Effective Dose

In certain aspects the present disclosure encompasses methods of treating or managing a disease or disorder, for example resulting from radiation exposure, which comprises administering to a patient or subject in need of such treatment or management a therapeutically effective amount of RLIP76 or a therapeutic combination of RLIP76 and another active agent, for example another radioprotective agent. In certain embodiments, such a compound or dosage unit comprising RLIP76 is referred to as an active agent. Use of the disclosed compositions in the manufacture of a medicament for treating or managing a disease or disorder is also contemplated. The present disclosure also encompasses compositions comprising a biologically or therapeutically effective amount of one or more cargo molecules for use in the preparation of a medicament for use in treatment or management of a disease or disorder.

As used herein, and unless otherwise indicated, the terms "treat," "treating," and "treatment" contemplate an action that occurs while a patient is suffering from a disease or disorder, that reduces the severity of one or more symptoms or effects of the disease or disorder, or a related disease or disorder. As used herein, and unless otherwise indicated, the terms "manage," "managing," and "management" encompass preventing, delaying, or reducing the severity of a recurrence of a disease or disorder in a patient who has already suffered from the disease or disorder. The terms encompass modulating the threshold, development, and/or duration of the disease or disorder, or changing the way that a patient responds to the disease or disorder.

As used herein, and unless otherwise specified, a "therapeutically effective amount" of a compound is an amount sufficient to provide any therapeutic benefit in the treatment or management of a disease or disorder, or to delay or minimize one or more symptoms associated with a disease or disorder. A therapeutically effective amount of a compound means an amount of the compound, alone or in combination with one or more other therapy and/or therapeutic agent, which provides any therapeutic benefit in the treatment or management of a disease or disorder, or related diseases or disorders. The term "therapeutically effective amount" can encompass an amount that cures a disease or disorder, improves or reduces a disease or disorder, reduces or avoids symptoms or causes of a disease or disorder, improves overall therapy, or enhances the therapeutic efficacy of another therapeutic agent.

Toxicity and therapeutic efficacy of the described compounds and compositions can be determined by standard pharmaceutical procedures in cell cultures or experimental animals, e.g., for determining the LD50 (the dose lethal to 50% of the population) and the ED50 (the dose therapeutically effective in 50% of the population). The dose ratio between toxic and therapeutic effects is the therapeutic index, expressed as the ratio LD50/ED50. Compounds that exhibit large therapeutic indices are preferred. Compounds that exhibit toxic side effects may be used in certain embodiments, however, care should usually be taken to design delivery systems that target such compounds preferentially to the site of affected tissue, in order to minimize potential damage to uninfected cells and, thereby, reduce side effects.

Data obtained from cell culture assays and animal studies can be used in formulating a range of dosages for use in humans. In certain aspects of the present disclosure, the dosages of such compounds lie within a range of circulating concentrations that include the ED50 with little or no toxicity. The dosage may vary within this range depending on the dosage form employed and the route of administration utilized. For any compound used in the disclosed methods, the therapeutically effective dose can be estimated initially from cell culture assays. A dose may be formulated in animal models to achieve a circulating plasma concentration range that includes the IC50 (*i.e.,* the concentration of the test compound that achieves a half-maximal inhibition of symptoms) as determined in cell culture. Such information can be used to more accurately determine useful doses in humans. Plasma levels may be measured, for example, by high performance liquid chromatography.

When therapeutic treatment is contemplated, the appropriate dosage may also be determined using animal studies to determine the maximal tolerable dose, or MTD, of a bioactive agent per kilogram weight of the test subject. In general, at least one animal species tested is mammalian. Those skilled in the art regularly extrapolate doses for efficacy and avoiding toxicity to other species, including human. Before human studies of efficacy are undertaken, Phase I clinical studies help establish safe doses. Additionally, the bioactive agent may be complexed with a variety of well established compounds or structures that, for instance, enhance the stability of the bioactive agent, or otherwise enhance its pharmacological properties (*e.g.,* increase *in vivo* half-life, reduce toxicity, *etc*.)*.*

In certain embodiments of the present disclosure, the effective dose of the composition or dosage unit can be in the range of about 14 mg/kg to about 0.01 mg/kg, about 14 mg/kg to about 0.025 mg/kg, about 14 mg/kg to about 0.05 mg/kg, about 14 mg/kg to about 0.1 mg/kg, about 14 mg/kg to about 0.25 mg/kg, about 14 mg/kg to about 0.5 mg/kg, about 14 mg/kg to about 1 mg/kg, about 14 mg/kg to about 2.5 mg/kg, about 14 mg/kg to about 5 mg/kg, about 5 mg/kg to about 0.01 mg/kg, about 2.5 mg/kg to about 0.01 mg/kg, about 1 mg/kg to about 0.01 mg/kg, about 0.5 mg/kg to about 0.01 mg/kg, about 0.25 mg/kg to about 0.01 mg/kg, about 0.1 mg/kg to about 0.01 mg/kg, about 0.05 mg/kg to about 0.01 mg/kg, about 0.025 mg/kg to about 0.01 mg/kg, about 5 mg/kg to about 0.025 mg/kg, about 2.5 mg/kg to about 0.05 mg/kg, about 1 mg/kg to about 0.1 mg/kg, about 0.5 mg/kg to about 0.25 mg/kg, or about 3 mg/kg to about 0.1 mg/kg, or so. Thus, in particular embodiments, the effective dose of the composition or dosage unit is about 0.01 mg/kg, about 0.025 mg/kg, about 0.05 mg/kg, about 0.075 mg/kg, about 0.1 mg/kg, about 0.25 mg/kg, about 0.5 mg/kg, about 0.75 mg/kg, about 1 mg/kg, about 2.5 mg/kg, about 3 mg/kg, about 5 mg/kg, about 7.5 mg/kg, about 10 mg/kg, about 11 mg/kg, about 12 mg/kg, about 13 mg/kg, about 14 mg/kg, or so.

### Kits

A typical kit comprises one or more dosage units of a composition comprising RLIP76, or a pharmaceutically acceptable salt, prodrug, solvate, hydrate, or stereoisomer thereof. In certain embodiments, a single dosage unit form of another agent, for example a radioprotective agent, may be used in combination with the disclosed compounds. Kits of the current disclosure can further comprise devices that are used to administer the active ingredients. Examples of such devices include, but are not limited to, syringes, drip bags, patches, and inhalers.

The disclosed kits can further comprise pharmaceutically acceptable vehicles that can be used to administer one or more disclosed compositions. For example, if a disclosed composition is provided in a solid form that is to be reconstituted for parenteral administration, the kit can comprise a sealed container of a suitable vehicle in which the disclosed composition can be dissolved to form a particulate-free sterile solution that is suitable for parenteral administration. Examples of pharmaceutically acceptable vehicles include, but are not limited to: Water for Injection USP; aqueous vehicles such as, but not limited to, Sodium Chloride Injection, Ringer's Injection, Dextrose Injection, Dextrose and Sodium Chloride Injection, and Lactated Ringer's Injection; water miscible vehicles such as, but not limited to, ethyl alcohol, polyethylene glycol, and polypropylene glycol; and nonaqueous vehicles such as, but not limited to, corn oil, cottonseed oil, peanut oil, sesame oil, ethyl oleate, isopropyl myristate, and benzyl benzoate. However, in specific embodiments, the disclosed formulations do not contain any alcohols or other co-solvents, oils or proteins.

The following examples are included to demonstrate preferred embodiments of the invention. It should be appreciated by those of skill in the art that the techniques disclosed in the examples which follow represent techniques discovered by the inventors to function well in the practice of the invention, and thus can be considered to constitute preferred modes for its practice. However, those of skill in the art should, in light of the present disclosure, appreciate that many changes can be made in the specific embodiments which are disclosed and still obtain a like or similar result without departing from the spirit and scope of the invention. The present invention is not to be limited in scope by the specific embodiments described herein, which are intended as single illustrations of individual aspects of the invention, and functionally equivalent methods and components are within the scope of the invention. Indeed, various modifications of the invention, in addition to those shown and described herein, will become apparent to those skilled in the art from the foregoing description. Such modifications are intended to fall within the scope of the appended claims.

### EXAMPLES

### EXAMPLE 1

### RADIATION PROTECTION BY RLIP76

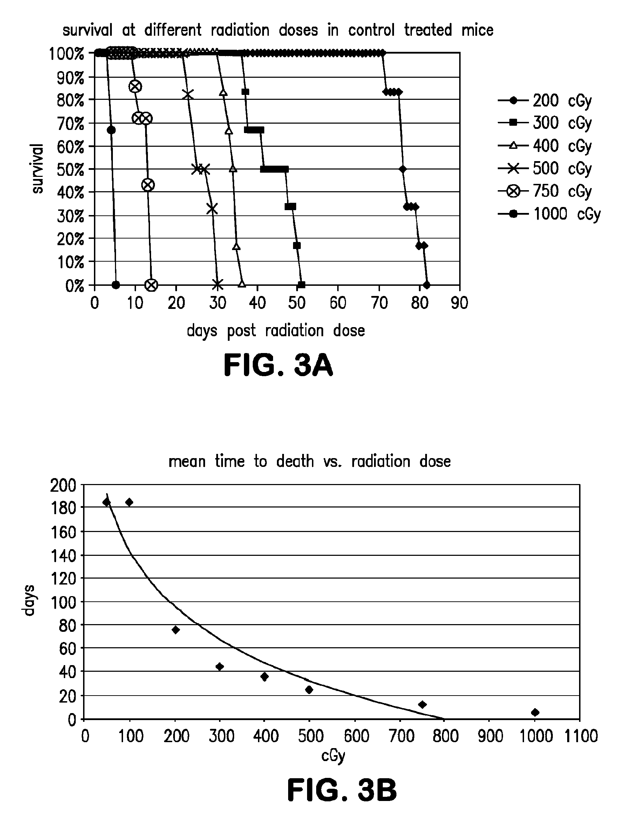

In all animal models, the relationship between radiation exposure and survival will vary depending upon experimental parameters, and so must be determined for each particular model with no treatment (control). Studies were conducted to show the effects of X irradiation on survival of mice. Baseline survival curves were obtained for mice from the C57/B16 strain treated with varying dosages of X-irradiation. **Figure 3A** shows survival at different radiation doses in control treated mice, while **Figure 3B** depicts the mean time to death versus radiation dose. Clearly, survival time diminishes with increasing dose of X-irradiation.

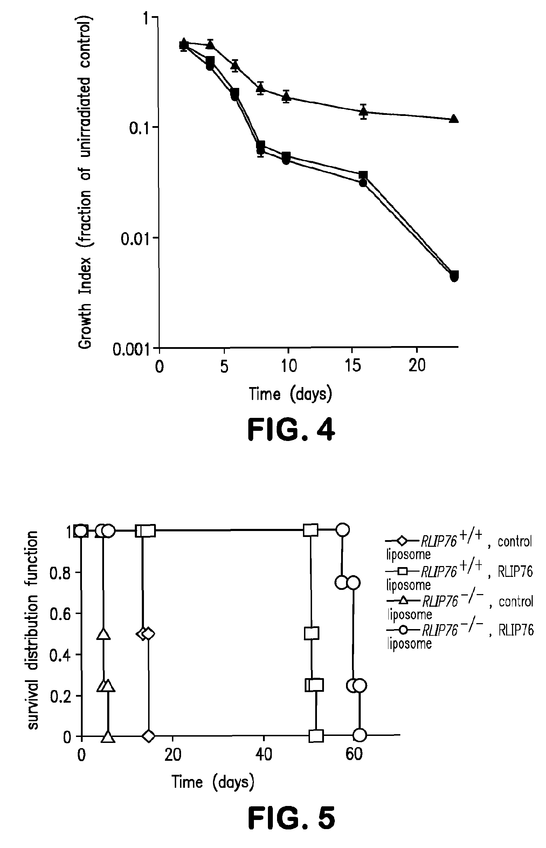

Surprisingly, administration of RLIP76 to wild-type mice improves their survival. Four wild-type mice were exposed to 1000 cGy and treated with control liposomes administered by i.p. injection (**Figure 4****,** diamonds) or 400 µg RLIP76 liposomes administered by i.p. injection (**Figure 4****,** squares) given on day +3. As shown, administration of the RLIP76 liposomes increased the survival of the wild-type mice.

### EXAMPLE 2

### RLIP76 AND RADIATION SENSITIVITY USING KNOCKOUT MICE

The finding that supplementation of RLIP76 levels above normal levels in mice is able to increase resistance of those mice to the toxic effects of radiation implies that RLIP76 functions as the "rate-limiting" step in this protective process. Therefore, increases in RLIP76 content may increase protection, using normal physiologic functions, in a dose and time responsive manner. Confirmation of this was established in studies investigating the effect of RLIP76 administration upon survival of irradiated mice under a variety of conditions.

As disclosed in U.S. Serial No. 11/741,447, C57B mice that carry both copies of the RIP1 (mouse version of RLIP76) gene (wild-type; +/+), one copy of the RIP1 gene (heterozygous; +/-), or no copies of the RIP1 gene (homozygous; -/-) were created using Cre-Lox technology that can selectively suppress genes (Lexicon Genetics, Incorporated, The Woodlands, TX). Western blot analysis of tissues from homozygous RIP1 knockout mice was performed after i.p. injection of RLIP76-liposomes. The effect of decreasing RIP1 expression on sensitivity to radiation was examined at 500 cGy, 750 cGy, and 1000 cGy of whole body X-irradiation of wild-type (+/+), heterozygous (+/-), and homozygous (-/-) RIP1 mice (6 mice per group), followed by monitoring for survival. As seen in Table 1, the wild-type (+/+) RIP1 mice had increased survival times at all radiation doses tested compared to the heterozygous (+/-) RIP1 mice, and the heterozygous (+/-) RIP1 mice had increased survival times at all radiation doses tested compared to the homozygous (-/-) RIP1 mice. Thus, increased radiation sensitivity was observed upon decreased RIP1 expression.

**Table 1**

| | Survival (Hours) | | |
|---|---|---|---|
| Radiation Dose (cGy) | Wild-Type (+/+) | Heterozygous (+/-) | Homozygous (-/-) |
| 500 | 648 ± 53 | 360 ± 28 | 264 ± 28 |
| 750 | 336 ± 30 | 168 ± 21 | 144 ± 12 |
| 1000 | 138 ± 14 | 19 ± 4 | 8 ± 3 |

If loss of RIP1 was the major determining factor in this acquired radiation sensitivity, replacement of this deficit should reverse radiation resistance. Therefore, a liposomal delivery system for providing recombinant human RLIP76 to the tissues of knockout animals was used. Methods for expressing recombinant human RLIP76 in *E. coli* and purifying the expressed protein to a high purity, >96% by amino acid composition analysis, and reconstituting its transport function in artificial liposomes were the same as those described in the art (Awasthi, et al., Biochemistry 39:9327-9334, 2000; incorporated herein by reference). Liposomes were prepared in sufficient quantities and administered via intraperitoneal (i.p.) injection to homozygous RIP1 mice.

This effect is seen at a higher radiation exposure **(****Figure 5****).** Four wild-type RIP1 mice were exposed to 750 cGy and treated with control liposomes administered by i.p. injection (**Figure 5****,** diamonds) or 400 µg RLIP76 liposomes administered by i.p. injection (**Figure 5****,** squares) given 12 hours after exposure, and four RIP -/- mice were exposed to 750 cGy and treated with control liposomes administered by i.p. injection (**Figure 5****,** triangles) or 400 µg RLIP76 liposomes administered by i.p. injection (**Figure 5****,** circles) given 12 hours after exposure. Clearly, administration of the RLIP76 liposomes increased the survival of the RIP1 1 mice, but it also dramatically increased the survival of the wild-type RIP1 mice. Remarkably, the RIP1-/- mice supplemented with RLIP76 had significantly improved survival as compared with even the RIP1+/+ mice. These findings conclusively demonstrate the radiation protective effects of RLIP76.

### EXAMPLE 3

### DOSE RESPONSE STUDIES

In order to investigate whether RLIP76 could be effective when delivered more than 24 hours after radiation exposure, a series of studies were conducted to explore the protective benefit of RLIP76 when given at varying doses, exposure levels, and times after exposure. All mice were C57BL6/albino strain, the strain most commonly used for radiation effect models. Exposure was delivered by whole body X-irradiation with a Varian Clinac Linear accelerator (2100C) at the Texas Cancer Center (Arlington, Texas). A 6-MeV photon beam was used at a rate of 400 cGy/minute. Mice were isolated to one side of their cage on top of 1.5 cm of superflab bolus and the field of treatment centered on them. Total dose was split into two fractions, anterior and posterior, by rotating the accelerator gantry 180 degrees. Unless otherwise specified, all experiments were performed on six mice per group. Notably, efficient protein delivery occurred through oral administration of RLIP76 proteoliposomes; therefore all experiments were performed with this route of administration.

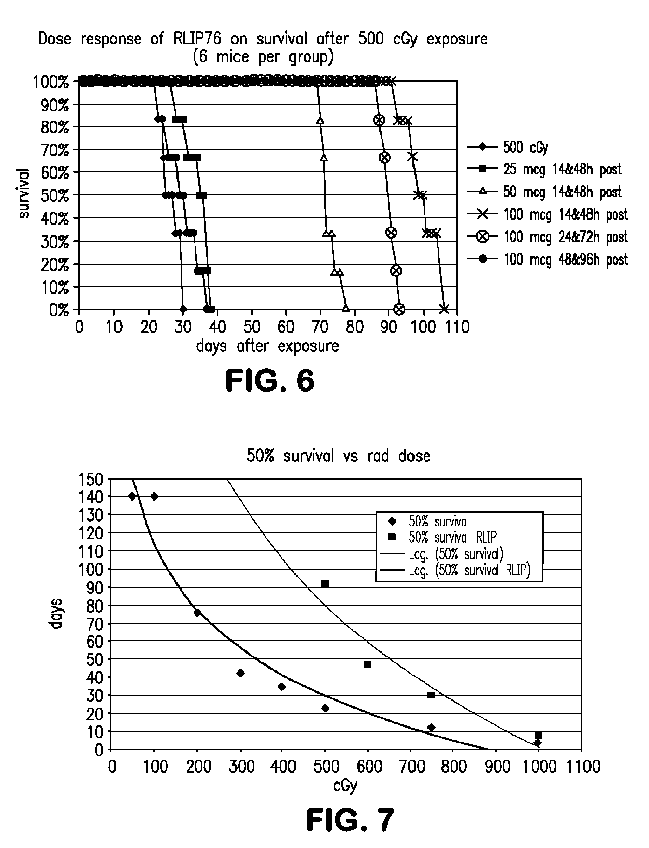

Dose and time dependency was investigated over a range of radiation exposures. Initially, six groups of wild-type mice were exposed to 500 cGy of radiation, and then treated with no RLIP76 proteoliposomes (control) or various dosages of RLIP76 proteoliposomes given at various times after the radiation exposure. **Figure 6** shows the dose response of administration of 25 µg of RLIP76 proteoliposomes given 14 and 48 hours after the radiation exposure (squares), 50 µg of RLIP76 proteoliposomes given 14 and 48 hours after the radiation exposure (triangles), 100 µg of RLIP76 proteoliposomes given 14 and 48 hours after the radiation exposure (X), 100 µg of RLIP76 proteoliposomes given 24 and 72 hours after the radiation exposure (*), or 100 µg of RLIP76 proteoliposomes given 48 and 96 hours after the radiation exposure (circles), compared to control mice that were not administered RLIP76 (diamonds). Significantly, even in mice with a normal complement of RLIP76 protein, a greatly increased resistance to radiation is conferred if additional RLIP76 protein is provided. Indeed, wild-type mice exposed to a dose of radiation that normally results in complete lethality by day 30 show a 200% increase in survival when given 2 doses of 100 micrograms of RLIP76 protein by oral administration **(****Figure 6****).** Surprisingly, a dose-reduction factor graph measuring 50% survival versus radiation dose of untreated and RLIP76 proteoliposome treated mice **(****Figure 7****)** shows that even waiting a full 24 hours after the radiation exposure before the initial dose is given still results in a remarkable dose-reduction factor ("DRF") of 1.7-1.8. The ability to administer RLIP76 more than 24 hours after radiation exposure and still obtain a therapeutic benefit is significant.

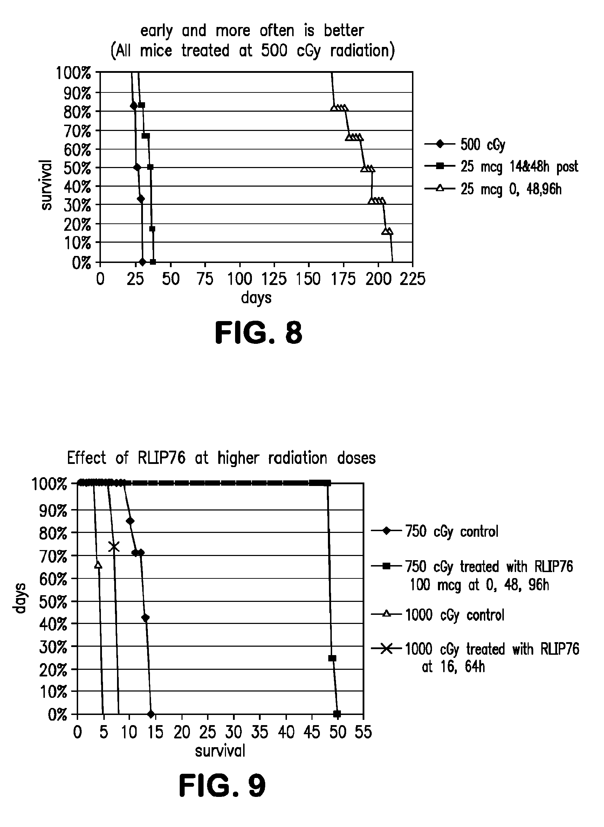

Three groups of wild-type mice were exposed to 500 cGy of radiation, and then one group was treated with no RLIP76 proteoliposomes (control; diamonds), one group was treated with 25 µg of RLIP76 proteoliposomes administered 14 and 48 hours after radiation exposure (squares), and one group was treated with 25 µg of RLIP76 proteoliposomes administered 0, 48, and 96 hours after radiation exposure (triangles). The results are shown in **Figure 8****,** and clearly demonstrate that the radioprotective effects of the RLIP76 proteoliposomes are improved by earlier and more frequent administration of RLIP76 proteoliposomes.

The radioprotective effect of RLIP76 proteoliposomes was also demonstrated at higher radiation doses. Two groups of wild-type mice were exposed to 750 cGy of radiation, and then one group was treated with no RLIP76 proteoliposomes (control; diamonds), and one group was treated with 100 µg of RLIP76 proteoliposomes administered 0, 48, and 96 hours after radiation exposure (squares). Additionally, two groups of wild-type mice were exposed to 1000 cGy of radiation, and then one group was treated with no RLIP76 proteoliposomes (control; triangles), and one group was treated with 100 µg of RLIP76 proteoliposomes administered 16 and 64 hours after radiation exposure (X). The results are shown in **Figure 9****,** and show that RLIP76 proteoliposomes provide excellent radiation protection at 750 cGy, and even some protection at 1000 cGy. In summary, RLIP76 offers substantial advantage over existing radioprotective candidates given its marked survival benefits, ability to be delivered orally, and at a significant delay after exposure.

### EXAMPLE 4

### RADIATION PROTECTION BY RLIP76 LIPOSOMES PLUS ANTI-OXIDANTS

Unprotected RLIP76 protein is susceptible to proteolysis, rendering administration of the bare protein challenging. In this study, RLIP76 was administered in the form of lipid encapsulated proteoliposomes. In order to reduce or prevent oxidative degradation while awaiting administration, the buffer in which RLIP76 was reconstituted into liposomes contained an antioxidant, for example butylated hydroxytoluene (BTH). One or more other antioxidants could also be added to the liquid encapsulated proteoliposomes comprising RLIP76. Of note, BHT has been reported in the scientific literature as having a radioprotective effect on its own. Liposomes have also been used to deliver candidate radiation countermeasure drugs, but the ability of liposomes themselves to offer protection is not clear from the literature.

Given that lipid-based delivery of RLIP76 may improve stability of the protein in a pharmaceutical formulation, a complex of RLIP76 protein, liposomes, and antioxidants (such as BHT) was generated, and designated TO-80Cx (the 80 refers to the mean size of the liposomes of 80 nm, which classifies them as intermediate sized vesicles). Further designations include TO-80LA which refers to the liposomes constituted in buffer with antioxidants (BHT) and TO-80L which refers to liposomes in buffer without antioxidants or RLIP76 protein. Next, these complexes of RLIP76 proteoliposomes with antioxidants such as BHT were tested to determine whether they can confer protection and/or therapeutic effect for radiation toxicity in excess of the effects of liposomes and BHT alone or in combination.

Overall survival of 14-week old CD2F1 male mice weighing an average of 30.0 g was measured after exposure to 9.25 Gy gamma radiation from a cobalt 60 source at a dose rate of 0.60 Gy/min. The mice were grouped into cohorts of 16 mice/cohort and received multiple doses via intraperitoneal administration with TO-80Cx 50 µg (weight of RLIP76 protein)/mouse, or individual drug components of the same volume/concentration, using multiple time regimens and compared to controls. Survival of the mice was studied for 30 days.

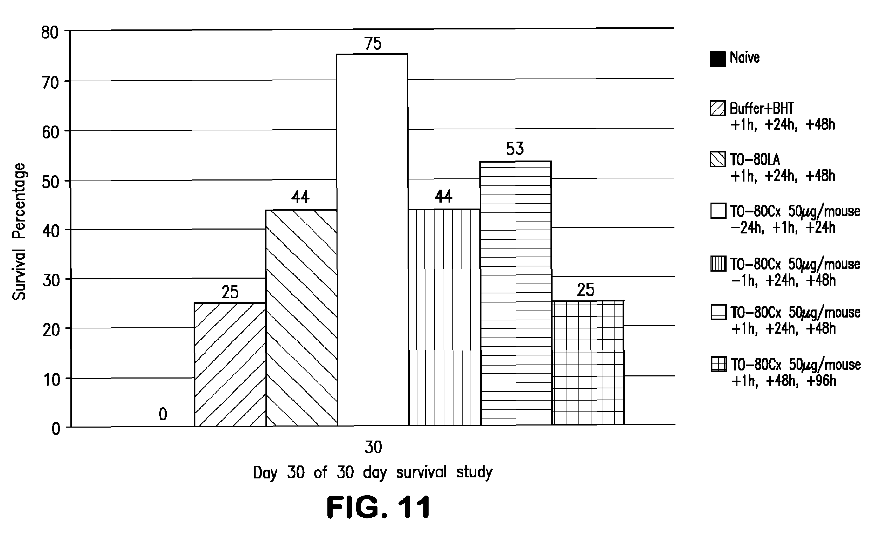

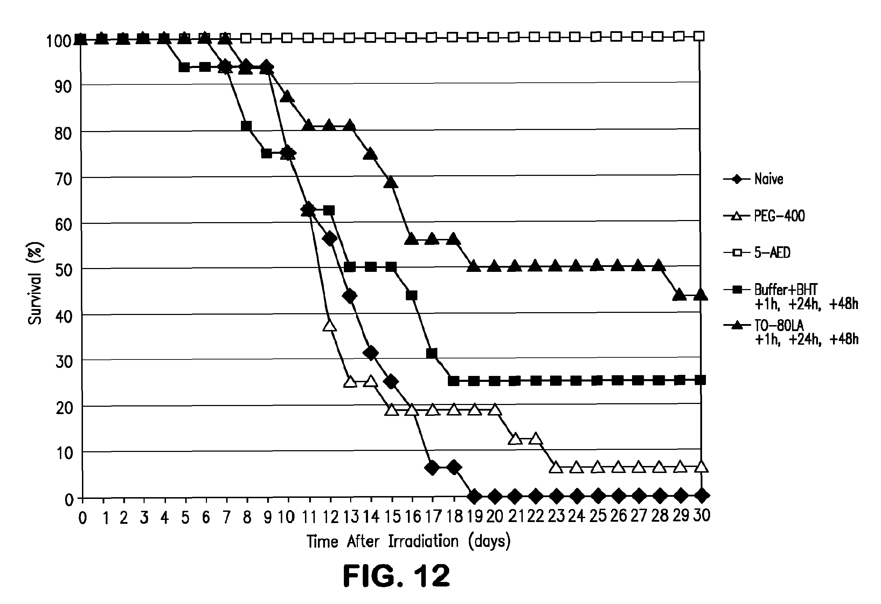

As shown in **Figure 10****,** maximum benefit was achieved with the full TO-80Cx complex given 24 hours prior to exposure. Lesser benefit in survival was seen if administration was delayed until around the time of exposure in this experiment, although other experiments have found greater effect even if administration is delayed by some hours. This data is also shown in **Figure 11****.** In this experiment, BHT containing buffer alone yielded a small effect (Buffer + BHT) and a combination of liposomes and BHT yielded a greater effect (TO-80LA). In this experiment, TO-80LA had effects similar to TO-80Cx given peri-exposure. In a previous set of experiments, however, TO 80LA was markedly inferior to the full TO-80Cx at a lower total radiation exposure dose, as shown in **Figure 13****.** Interestingly, as shown in **Figure 12** a comparison of different delivery vehicles without RLIP76 showed that TO 80LA has some protective effect compared to BHT containing buffer alone, suggesting that the liposomes themselves may have some radioprotective effect.

Thus, each active component of TO-80Cx has some effect as a radioprotectant. However, maximum effect is seen with the full complex. The specific contribution in quantitative terms for liposomes or liposomes plus BHT remains variable and may depend upon the level of radiation exposure.

### EXAMPLE 5

### RLIP76 AS A SPACE RADIATION COUNTERMEASURE

As the nature of space exploration has shifted from temporary missions to longer term projects (such as the International Space Station), the risks of these explorations has also shifted from purely mechanical issues to concerns about the inherent dangers of space itself. Chief among these are concerns about prolonged exposure to radiation. Space is filled with radiation, including electromagnetic radiation (X-rays and gamma rays) and cosmic rays (protons and heavy ions) that are normally blocked or attenuated at the Earth's surface. Particle radiations also produce secondary radiations such as neutrons and gamma rays when they interact with matter. This fact is relevant to problems of shielding spacecraft and their contents; not only is shielding heavy, it could generate more dangerous radiation.

A typical human on Earth experiences less than 5 x 10-3 Gy in a year. In outer space, solar flares can produce exposure levels up to 3 Gy, but for the most part exposures are much less. However, ionizing radiations other than x-rays, gamma-rays and energetic electrons (including β particles) cause more biological damage per unit energy absorbed than do these radiations, and so measurements are corrected by a factor called the Relative Biological Effectiveness (RBE). The RBE concept is important in space radiation health, particularly since damage accumulates with continued exposure during the entire time a person is outside the earth's atmosphere. The risks presented to space travelers by these radiations include cancer due to chronic proton and cosmic-ray exposure, immune failure due to high-dose solar proton storms, and possible neurological effects caused by single tracks of cosmic-ray heavy nuclei in neuronal tissue. These effects include cognitive dysfunction and retinal flashes (already described in one Apollo astronaut).

The evidence of the efficacy of RLIP76 is supported by experimental data, as detailed above. Mice genetically deficient in RLIP76 protein are exquisitely sensitive to a wide range of radiation exposure levels, and this sensitivity can be reversed with supplementation of RLIP76 protein by either intravenous or oral administration. Significantly, even in mice with a normal complement of RLIP76 protein a greatly increased resistance to radiation is conferred if additional RLIP76 protein is provided. Indeed, wild-type mice exposed to a dose of radiation that normally results in complete lethality by day 30 show a 200% increase in survival when given 2 doses of 100 micrograms of RLIP76 protein by oral administration. As expected, the earlier the RLIP76 is administered, the greater the effect, but even waiting a full 24 hours or more after the radiation exposure before the initial dose is given still results in significant rescue.

Perhaps most significantly, though, is the finding that RLIP76 encapsulated in proteoliposomes crosses the blood-brain barrier even with oral administration. Thus, the protective effect of RLIP76 would extend to central nervous system tissue such as the hippocampus, localized as the site of much of the neurologic damage seen in rats bombarded by heavy ions. Thus, the combination of mechanism of action, ease of administration and anatomic delivery makes RLIP76 an attractive candidate as a Space Radiation Countermeasure.

All of the compositions and/or methods disclosed and claimed herein can be made and executed without undue experimentation in light of the present disclosure. While the compositions and methods of this invention have been described in terms of preferred embodiments, it will be apparent to those of skill in the art that variations may be applied to the compositions and/or methods and in the steps or in the sequence of steps of the method described herein without departing from the concept, spirit and scope of the invention. More specifically, it will be apparent that certain agents which are both chemically and physiologically related may be substituted for the agents described herein while the same or similar results would be achieved. All such similar substitutes and modifications apparent to those skilled in the art are deemed to be within the spirit, scope and concept of the invention as defined by the appended claims. Aspects and features of the present disclosure are now set out in the following numbered clauses, which contain the subject matter of the claims of the parent application as filed.
1. A method of treating the effects of radiation exposure in a subject in need of such treatment, comprising administering an effective amount of RLIP76 protein or an active fragment thereof to the organism more than 24 hours after the radiation exposure.
2. The method of claim 1, wherein the subject is a mammalian subject.
3. The method of claim 1, wherein the radiation is ionizing radiation.
4. The method of claim 3, wherein the ionizing radiation is selected from the group consisting of X-radiation, γ-radiation, energetic electron radiation, ultraviolet radiation, thermal radiation, cosmic radiation, electromagnetic radiation, nuclear radiation, or a combination thereof.
5. The method of claim 1, wherein the RLIP76 protein or an active fragment thereof is administered to the subject more than 36 hours after the radiation exposure.
6. The method of claim 1, wherein the RLIP76 protein or an active fragment thereof is administered to the subject more than 48 hours, 60 hours, 72 hours, 84 hours, or 96 hours after the radiation exposure.
7. The method of claim 1, wherein the RLIP76 protein or an active fragment thereof is administered with a liposome or a proteoliposome.
8. The method of claim 1, wherein the RLIP76 protein or an active fragment thereof is administered in one or more doses to the subject.
9. The method of claim 4, wherein the energetic electron radiation is β-particle radiation.
10. The method of claim 1, wherein the radiation is proton or heavy ion radiation.
11. The method of claim 1, further comprising administering a second radiation protection agent to the subject.
12. The method of claim 11, wherein the second radiation protection agent is a free radical scavenger, an antioxidant, or a superoxide dismutase analog.
13. A method of treating the effects of exposure to radiation in an subject in need of such treatment, comprising administering (a) at least a first dose of an effective amount of RLIP76 protein or an active fragment thereof to the subject within 24 hours after the radiation exposure, and (b) at least a second dose of an effective amount of RLIP76 protein or an effective portion thereof to the subject more than 24 hours after radiation exposure.
14. The method of claim 1 or 13, wherein the subject is a human.
15. The method of claim 13, wherein the radiation is ionizing radiation.
16. The method of claim 15, wherein the ionizing radiation is selected from the group consisting of X-radiation, γ-radiation, energetic electron radiation, ultraviolet radiation, thermal radiation, cosmic radiation, electromagnetic radiation, nuclear radiation, or a combination thereof.
17. The method of claim 13, wherein the first dose is administered at about the time of the exposure to the ionizing radiation.
18. The method of claim 13, wherein the RLIP76 protein or an effective portion thereof is administered with a liposome or a proteoliposome.
19. The method of claim 13, further comprising administering to the subject one or more additional doses of an effective amount of RLIP76 protein or an effective portion thereof to the subject within 24 hours after the radiation exposure, more than 24 hours after radiation exposure, or both.
20. The method of claim 13, wherein the first dose and the second dose comprise about the same amount of the RLIP76 protein or an active fragment thereof.
21. The method of claim 13, wherein the first dose and the second dose comprise different amounts of the RLIP76 protein or active fragment thereof.
22. A composition comprising RLIP76 protein or an active fragment thereof and a second radiation protection agent.
23. The composition of claim 22, wherein the second radiation protection agent is a free radical scavenger, an antioxidant, or a superoxide dismutase analog.
24. The composition of claim 22, wherein the composition is administered with a liposome or a proteoliposome.
25. The composition of claim 23, wherein the antioxidant is butylated hydroxytoluene (BTH).
26. The method of claim 1 or 13, wherein the RLIP76 protein or active fragments thereof is administered from one time to ten times after 24 hours post-radiation.
27. The method of claim 1 or 13, wherein the RLIP76 protein or active fragments thereof is administered at least thee times after 24 hours post-radiation.
28. The method of claim 1 or 13, wherein the RLIP76 protein or active fragments thereof is administered once within 24 hours post-radiation and one or more times after 24 hours post-radiation.
29. The method of claim 1 or 13, wherein the RLIP76 protein or active fragments thereof is administered one or more times between 25 hours and three months post-radiation.
30. The method of claim 1 or 13, wherein the RLIP76 protein or active fragments thereof is administered at a dosage of at least 0.01 µg/kg body weight.
31. The method of claim 1 or 13, wherein the RLIP76 protein or active fragments thereof is administered at a dosage of between about 0.01 µg/kg body weight and about 100 mg/kg body weight.
32. The method of claim 1 or 13, wherein the RLIP76 protein or active fragments thereof is administered via an administration route selected from the group consisting of intravenous, intramuscular, subcutaneous, intraperitoneal, and oral administration.
33. The method of claim 1 or 13, wherein the radiation exposure is at least 2 Gy.
34. The method of claim 1 or 13, wherein the radiation exposure is between about 2 Gy and about 100 Gy.

## Claims

1. RLIP76 protein for use in treating the effects of radiation exposure in a subject in need of such treatment, comprising administering more than one dose of the RLIP76 protein to the subject more than 24 hours after the radiation exposure, wherein the subject has not been previously administered with RLIP76 protein prior to radiation exposure.

2. The RLIP76 protein for use as claimed in claim 1, wherein the subject is a mammal, optionally a human.

3. The RLIP76 protein for use as claimed in claim 1 or claim 2, wherein the radiation is ionising radiation, or wherein the radiation is selected from the group consisting of X-radiation, γ-radiation, energetic electron radiation β-particle radiation, ultraviolet radiation, thermal radiation, cosmic radiation, electromagnetic radiation, nuclear radiation, or a combination thereof, or wherein the radiation is proton or heavy ion radiation.

4. The RLIP76 protein for use as claimed in claim 1, wherein the RLIP76 protein is administered to the subject more than 36 hours, more than 48 hours, more than 60 hours, more than 72 hours, more than 84 hours, and/or more than 96 hours after the radiation exposure.

5. The RLIP76 protein for use as claimed in claim 1, wherein a first dose and a second dose of RLIP76 protein are administered.

6. The RLIP76 protein for use as claimed in claim 5, wherein the doses comprise about the same amount of the RLIP76 protein or the doses comprise different amounts of the RLIP76 protein.

7. The RLIP76 protein for use as claimed in claim 1, wherein the protein is administered from two times to ten times after 24 hours post-radiation, or wherein the RLIP76 protein is administered at least three times after 24 hours post-radiation.

8. The RLIP76 protein for use as claimed in claim 1, wherein the RLIP76 protein is administered between 25 hours and three months post-radiation.

9. The RLIP76 protein as claimed in claim 1, wherein the protein is administered as a dosage of at least 0.01 µg/kg body weight, or wherein the RLIP76 protein is administered at a dosage of between 0.01 µg/kg body weight and 100 mg/kg body weight.

10. The RLIP76 protein for use as claimed in claim 1, wherein the protein is administered via an administration route selected from the group consisting of intravenous, intramuscular, subcutaneous, intraperitoneal, and oral administration.

11. The RLIP76 protein for use as claimed in claim 1, wherein the radiation exposure is at least 2 Gy, or wherein the radiation exposure is between 2 Gy and 100 Gy.

12. The RLIP76 protein for use as claimed in claim 1, wherein the RLIP76 protein is administered within a liposome or a proteoliposome.

13. Use of RLIP76 protein in the manufacture of a medicament for treating the effects of radiation exposure in a subject, wherein more than one dose of RLIP76 protein is administered more than 24 hours after the radiation exposure, wherein the subject has not previously been administered with RLIP76 protein prior to radiation exposure.
